# EUROPÄISCHE PATENTSCHRIFT

(11) **EP 4 525 812 B1**
(45) Veröffentlichungstag und Bekanntmachung des Hinweises auf die Patenterteilung: **12.11.2025**
(21) Anmeldenummer: 23738613.1
(22) Anmeldetag: 23.06.2023
(51) Int. Cl.: A61H 1/02, A61F 5/042

(54) **EXTENSIONSVORRICHTUNGSANORDNUNG**
EXTENSION DEVICE ARRANGEMENT
DISPOSITIF D'EXTENSION

(30) Priorität: 23.06.2022 DE 202022103507 U
(43) Veröffentlichungstag der Anmeldung: 26.03.2025
(73) Patentinhaber: El Kattan, Ali, 44388 Dortmund (DE)
(72) Erfinder: El Kattan, Ali, 44388 Dortmund (DE)
(74) Vertreter: Osterhoff, Utz
(86) Internationale Anmeldenummer: PCT/DE2023/100479
(87) Internationale Veröffentlichungsnummer: WO 2023/246987

(56) Entgegenhaltungen:
- FR-A1- 2 708 195
- US-A- 4 890 605
- US-A- 5 129 881
- US-A1- 2010 280 549

## Beschreibung

Die vorliegende Erfindung betrifft eine Extensionsvorrichtungsanordnung gemäß den Merkmalen von Anspruch 1.

Extensionsvorrichtungen, auch Traktionsvorrichtungen genannt, sind im Stand der Technik bekannt und ermöglichen die Durchführung einer Extensionsbehandlung, bei der die Wirbelsäule oder andere Gelenke eines Patienten gezielt gestreckt werden. Gelenkpartner werden hierbei auseinandergezogen, so dass die Gelenkflächen entlastet werden und die umliegende Muskulatur gedehnt wird. Hierdurch verbessert sich die Beweglichkeit und Schmerzen werden reduziert. Die Extension dient ebenfalls der gezielten Entlastung eines gereizten Nervs. Auch Spastiken bei Schlaganfallpatienten können mittels Extensionsvorrichtungen behandelt werden.

Die Behandlung selbst kann mit oder ohne Hilfsmittel erfolgen. Bei der Behandlung ohne Hilfsmittel werden die Körperteile bzw. Gelenke des Patienten direkt durch einen Therapeuten gestreckt. Dies hat allerdings zwei wesentliche Nachteile. Zum einen können Extensionsbehandlungen mehrere Minuten bis Stunden dauern, so dass die Behandlung für den ausführenden Therapeuten sehr kräftezehrend ist. Der zweite Nachteil ist, dass die vom Therapeuten aufgebrachte Kraft nicht gemessen und nicht exakt sowie reproduzierbar aufgebracht werden kann. Insbesondere bei einer sich über mehrere Wochen oder Monate erstreckenden Behandlung können so beispielsweise mögliche Fortschritte nicht genau dokumentiert werden.

Für die Behandlung mit Hilfsmitteln werden in der Regel sogenannte Extensionsliegen bzw. Extensionstische eingesetzt. Üblicherweise wird ein Körperteil des Patienten direkt an der Extensionsliege fixiert, während ein anderes Körperteil über Seilzüge und einen computergesteuerten Motor gestreckt wird. Die Liege ist speziell für die Extensionsbehandlung ausgelegt und der Motor, die Seilzüge sowie die Befestigungsmittel sind fest an der Liege angebracht, so dass nur eine stationäre Behandlung eines Patienten möglich ist. Die Behandlung eines bettlägerigen Patienten auf einer solchen Liege ist somit nicht möglich oder mit einer erheblichen Belastung für den Patienten verbunden. Auch eine Behandlung in häuslicher Umgebung kann mit handelsüblichen Extensionsliegen nicht durchgeführt werden. Ein weiterer Nachteil gängiger Extensionsliegen ist, dass diese sehr teuer in der Anschaffung sind.

US 2010/280549 A1 offenbart eine Extensionsvorrichtungsanordnung aufweisend eine Liegevorrichtung mit einer Liegefläche und einen die Liegevorrichtung außen umgreifenden Rahmen, wobei der Rahmen einen unteren Längsbalken und einen oberen Längsbalken aufweist, die in ihren jeweiligen Endbereichen über einen ersten Pfosten und einen zweiten Pfosten miteinander gekoppelt sind, wobei an dem ersten Pfosten eine Zugvorrichtung angeordnet ist, so dass ein auf der Liegefläche befindlicher Patient jeweils an einem Körperteil oder Körperabschnitt über ein jeweiliges Koppelmittel mit der Zugvorrichtung verbunden ist.

Ausgehend vom Stand der Technik ist daher die Aufgabe der vorliegenden Erfindung, eine kostengünstige Alternative zu einer klassischen Extensionsliege bereitzustellen, die mit einer beliebigen Liegevorrichtung kombinierbar ist und einfach auf- und wieder abgebaut werden kann.

Die Aufgabe wird mit einer Extensionsvorrichtungsanordnung mit den Merkmalen des Anspruchs 1 gelöst.

Vorteilhafte Ausführungsvarianten sind Gegenstand der abhängigen Ansprüche. Die vorliegende Erfindung betrifft eine Extensionsvorrichtungsanordnung mit einer Liegevorrichtung und einen die Liegevorrichtung außen umgreifenden Rahmen.

Die Liegevorrichtung weist eine Liegefläche auf und ist bevorzugt als Bett, besonders bevorzugt als Krankenbett ausgebildet.

Der Rahmen ist aus einem unteren Längsbalken und einem oberen Längsbalken gebildet, die in ihren jeweiligen Endbereichen über einen ersten und einen zweiten Pfosten miteinander gekoppelt sind. Der Rahmen wird mit dem unteren Längsbalken unterhalb der Liegevorrichtung angeordnet.

Erfindungsgemäß ist im Höhenabschnitt der Liegefläche an dem ersten Pfosten eine Zugvorrichtung angebracht und an dem zweiten Pfosten ein Gegenhalter angeordnet. Ein auf der Liegefläche befindlicher Patient ist jeweils an einem Körperteil oder Körperabschnitt über ein Koppelmittel mit der Zugvorrichtung und mit dem Gegenhalter verbunden.

Die erfindungsgemäße Extensionsvorrichtungsanordnung zeichnet sich nunmehr dadurch aus, dass durch die Zugvorrichtung zwischen den Körperteilen oder Körperabschnitten des Patienten eine im Wesentlichen parallel zur Liegefläche wirkende Zugkraft aufgebracht ist.

Die Extensionsvorrichtungsanordnung bietet den Vorteil, dass sie an einer beliebigen Liegevorrichtung aufgebaut werden kann. Beispielsweise muss ein Patient sein Krankenbett nicht verlassen und kann vor Ort behandelt werden. Dies ist insbesondere für bettlägerige Patienten ein entscheidender Vorteil, da auch kein Umbetten erforderlich ist. Der Rahmen der Extensionsvorrichtungsanordnung kann für die Dauer der Behandlung, die mehrere Tage bis mehrere Monate andauern kann, an der Liegevorrichtung verbleiben. Nach Beendigung der Behandlung kann der Rahmen zusammengebaut und an einer anderen Liegevorrichtung angeordnet werden.

Durch den einfachen Aufbau der Extensionsvorrichtung sind die Herstellungsund Anschaffungskosten im Vergleich zu herkömmlichen Extensionsliegen deutlich geringer.

Erfindungsgemäß ist der obere Längsbalken lösbar mit den Pfosten verbunden. Dies hat den Vorteil, dass dieser nur während der eigentlichen Extensionsbehandlung im Rahmen fixiert werden muss, um die Stabilität des Rahmens während der Streckung zu gewährleisten. In der übrigen Zeit kann der obere Längsbalken entfernt werden, so dass dieser das Blickfeld des Patienten und auch sonstige erforderliche Behandlungen nicht beeinträchtigt.

Vorzugsweise ist auch der untere Längsbalken lösbar mit den Pfosten gekoppelt, so dass auch diese schnell auseinandergebaut und verstaut werden können. So kann die Extensionsvorrichtung schnell an einer Liegevorrichtung nachgerüstet werden.

Um die Standfestigkeit des Rahmens zu erhöhen sind vorzugsweise Standfüße am unteren Längsbalken angeordnet. Diese sind insbesondere aus kürzeren Pfostenabschnitten ausgebildet, die senkrecht zur unteren Längsbalkenrichtung beidseitig an den Endbereichen des unteren Längsbalkens angeordnet sind.

Als Koppelmittel zur Verbindung des Körperteils bzw. Körperabschnitts des Patienten mit der Zugvorrichtung werden insbesondere Manschetten verwendet, die über ein Verbindungsmittel, bevorzugt Drahtseile, mit der Zugvorrichtung verbunden sind. Die Manschetten sind insbesondere dazu geeignet, an den Fußgelenken eines Patienten angebracht zu sein. Bevorzugt weisen die Manschetten Ösen auf, über die sie mit dem Verbindungsmittel gekoppelt sind.

Die Zugvorrichtung selbst ist vorteilhafterweise aus einer Handwinde oder einer Elektroseilwinde ausgebildet. Dies ermöglicht die Übertragung einer definierten Kraft auf den Patienten, ohne eine Belastung für den behandelnden Therapeuten darzustellen.

Das Koppelmittel zur Verbindung des Körperteils bzw. Körperabschnitts mit dem Gegenhalter ist insbesondere aus einem Geschirr ausgebildet, welches ebenfalls über Verbindungsmittel, bevorzugt Drahtseile, mit dem Gegenhalter verbunden ist. Auch hier weist das Geschirr insbesondere zwei Ösen zur Kopplung mit dem Verbindungsabschnitt auf. Um die Stabilität dieser Kopplung weiter zu erhöhen können auch mehr als zwei Ösen an dem Geschirr angeordnet sein.

Das Geschirr ist insbesondere an der Hüfte des Pateinten angeordnet. Bei gleichzeitiger Anordnung der Manschetten an den Fußgelenken des Patienten und aufbringen einer Zugkraft durch die Zugvorrichtung, werden die Beine des Patienten parallel zur Liegefläche gestreckt. Dies ist insbesondere vorteilhaft für die Behandlung von Spastiken bei Schlaganfällen.

In einer weiteren Ausführungsvariante ist das Geschirr im Brustbereich des Patienten angeordnet. Bei gleichzeitiger Anordnung der Manschetten an den Fußgelenken des Pateinten und aufbringen einer Zugkraft durch die Zugvorrichtung, wird so insbesondere die Wirbelsäule gestreckt und entlastet.

Das Geschirr ist in einer alternativen Ausführungsvariante im Hals/Kopf Bereich des Pateinten angeordnet. So kann insbesondere der obere Wirbelsäulenbereich behandelt werden.

Vorzugsweise sind die Zugvorrichtung und der Gegenhalter höhenverstellbar an den Pfosten abgeordnet. Dies hat den Vorteil, dass die Vorrichtung an Liegevorrichtungen mit unterschiedlichen Abmessungen anpassbar ist.

Bevorzugt weist die Zugvorrichtung einen Kraftmesser und/oder Kraftbegrenzer auf. Der Kraftmesser dient zum Ablesen der von der Zugvorrichtung aufgebrachten Zugkraft, so dass eine definierte Kraft auf den Körper des Patienten aufgebracht werden kann. Der Kraftbegrenzer hat die Funktion, die maximal auf den Körper des Patienten aufgebrachte Kraft zu limitieren. Ist eine vorab festgesetzte Maximalkraft erreicht, kann diese von der Zugvorrichtung nicht weiter erhöht werden. Muskeln oder Gelenke des Patienten können so nicht in Mitleidenschaft gezogen werden.

Vorzugsweise weist das Verbindungsmittel der Zugvorrichtung einen Längenindikator auf. Anhand der Zusammenschau der aufgebrachten Kraft und der gestreckten Länge ist beispielsweise der Behandlungsfortschritt des Patienten über mehrere Sitzungen erfassbar. Bleibt die aufgebrachte Kraft konstant und die gestreckte Länge vergrößert sich, wurde der Bewegungsradius des behandelten Körperabschnitts erhöht.

Der Gegenhalter weist bevorzugt eine Traverse auf. Die Traverse weist an ihren jeweiligen Endbereichen Ösen auf, die über das Verbindungsmittel an dem Koppelmittel angeordnet werden.

Ferner kann der Gegenhalter an seinen jeweiligen Enden über die Liegevorrichtung hinausragen. Dies hat den Vorteil, dass die Verbindungsmittel seitlich an einem Fußteil der Liegevorrichtung vorbeigeführt werden können. Hierfür ist der Gegenhalter insbesondere zwischen 0.8 und 1,3 m breit.

Der Rahmen selbst ist bevorzugt aus Holz, Metall, Leichtmetall oder Kunststoff ausgebildet.

Weitere Vorteile, Merkmale, Eigenschaften und Aspekte der vorliegenden Erfindung sind Bestandteil der folgenden Beschreibung. Bevorzugte Ausgestaltungsvarianten sind in den schematischen Figuren dargestellt. Diese dienen dem einfachen Verständnis der Erfindung. Es zeigen:
Figur 1 eine Seitenansicht der Extensionsvorrichtungsanordnung und
Figur 2 eine Draufsicht der Extensionsvorrichtungsanordnung.

In den Figuren werden für gleiche oder ähnliche Gegenstände dieselben Bezugszeichen verwendet, auch wenn eine wiederholte Beschreibung aus Vereinfachungsgründen entfällt.

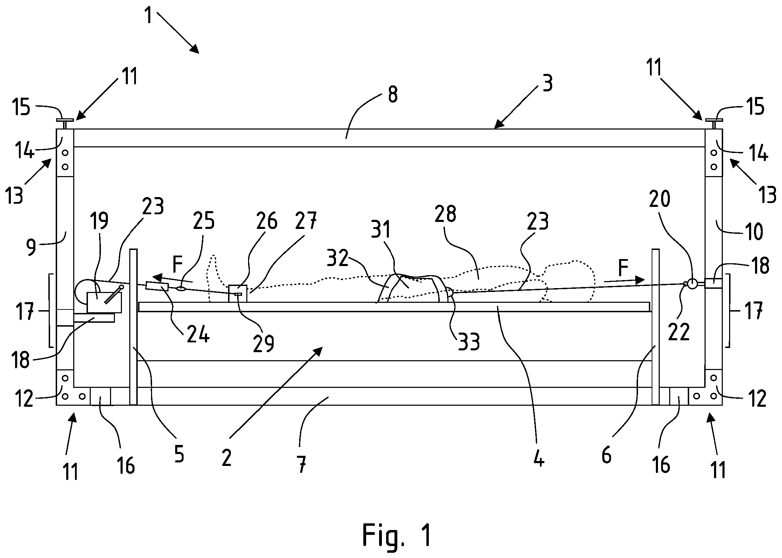

Figur 1 zeigt eine Seitenansicht einer erfindungsgemäßen Extensionsvorrichtungsanordnung 1, die eine Liegevorrichtung 2 und einen Rahmen 3 aufweist.

Die Liegevorrichtung 2 ist ferner aus einer Liegefläche 4, einem Fußteil 5 und einem Kopfteil 6 ausgebildet.

Der Rahmen 3 weist einen unteren Längsbalken 7 und einen oberen Längsbalken 8 auf, die über einen ersten Pfosten 9 und einen zweiten Pfosten 10 an ihren jeweiligen Endbereichen 11 miteinander verbunden sind. Der untere Längsbalken 7 ist an seinen jeweiligen Endbereichen 11 durch Winkelverbinder 12 mit den beiden Pfosten 9, 10 lösbar gekoppelt.

An den oberen Enden 13 der Pfosten 9, 10 sind jeweils Balkenverbinder 14 angeordnet. Diese sind so ausgeführt, dass der obere Balken 8 in die Balkenverbinder 14 eingesetzt werden kann. Für eine stabile und sichere Verbindung wird der obere Balken 8 über Hammerkopfschrauben 15 an seinen jeweiligen Endbereichen 11 an dem Balkenverbinder 14 lösbar fixiert.

Der Rahmen 3 ist so angeordnet, dass der untere Längsbalken 7 unterhalb der Liegevorrichtung 2 positioniert ist. Der untere Längsbalken 7 verläuft somit zwischen den unteren Füßen des Kopf- und Fußteils 5, 6 der Liegevorrichtung 2.

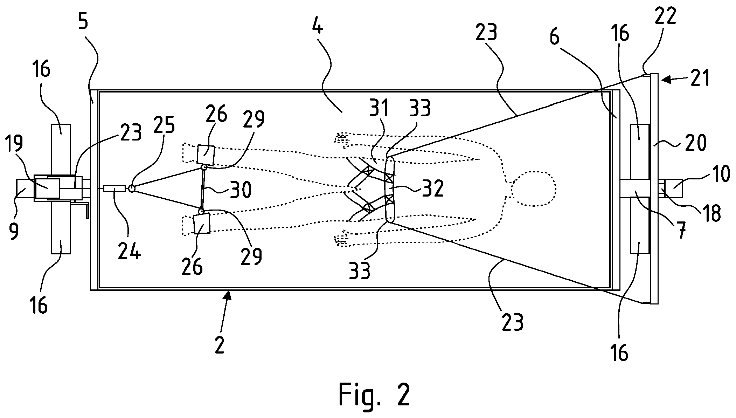

An dem untern Längsbalken 7 sind jeweils vor den Winkelverbindern 12 beidseitig Stützpfosten 16 angeordnet, die die Standsicherheit des Rahmens 3 gewährleisten. Siehe hierzu auch Figur 2.

Im Höhenabschnitt 17 der Liegefläche 4 ist am ersten Pfosten 9 eine Halterung 18 angeordnet. Auf der Halterung 18 ist eine Zugvorrichtung 19 positioniert.

Auch beim zweiten Pfosten 10 ist im Höhenabschnitt 17 der Liegefläche 4 eine Halterung 18 vorgesehen. An dieser ist ein Gegenhalter 20 abgeordnet, an dessen jeweiligen Endbereichen 21 jeweils eine Öse 22 angeordnet ist. Siehe hierzu auch Figur 2.

An der Zugvorrichtung 19 ist ein Verbindungsmittel 23 in Form eines Drahtseils eingespannt, welches zunächst bis zu einem Kombigerät 24 aus Kraftmesser und Kraftbegrenzer verläuft. Hierzu ist das Verbindungsmittel 23 durch das gitterförmige Fußteil 5 der Liegevorrichtung 2 geführt. Vom Kombigerät 24 verläuft das Verbindungsmittel 23 zu einem Teilungselement 25. Von dem Teilungselement 25 verlaufen wiederum zwei Verbindungsmittel 23 jeweils zu einem unteren Koppelmittel 26. Die unteren Koppelmittel 26 sind jeweils an einem ersten Körperteil 27 eines Patienten 28 angebracht, wobei es sich in dieser Ausführungsvariante um die Fußgelenke des Patienten 28 handelt. Die unteren Koppelmittel 26 weisen für die Verbindung mit den Drahtseilen 23 Ösen 29 auf.

Durch diese Y-förmige Anordnung der Verbindungsmittel 23 ist sichergestellt, dass auf beide unteren Koppelmittel 26 die gleiche Kraft F in eine im Wesentlichen parallel zur Liegefläche 4 verlaufenden Richtung aufgebracht wird.

Zwischen den Ösen 29 ist zusätzlich ein Stabilisierungselement 30 angeordnet, das sicherstellt, dass die unteren Koppelmittel 26 bei Aufbringung der Kraft F auf Abstand gehalten werden. Der Patient 28 wird so zusätzlich stabilisiert und eine Drehbewegung des Patienten 28 verhindert.

An einem zweiten Körperteil 31 des Patienten 28 ist ein oberes Koppelmittel 32 angeordnet, welches in diesem Ausführungsbeispiel ein Geschirr ist. Dieses besteht aus einer Vielzahl miteinander verbundener Gurte, welche den Hüftbereich des Patienten 28 umgreifen.

An dem oberen Koppelmittel 32 sind ferner zwei seitliche Ösen 33 angeordnet, die über Verbindungsmittel 23 mit den Ösen 22 des Gegenhalters 20 gekoppelt sind.

Der Gegenhalter 20 ragt an seinen jeweiligen Endbereichen 21 über das Kopfteil 6 der Liegevorrichtung 2 hinaus. Die Verbindungsmittel 23 verlaufen so seitlich an dem Kopfteil 6 vorbei. Die Koppelmittel 26, 32 und die dazugehörigen Verbindungsmittel 23 sind somit alle auf einer im Wesentlichen zur Liegefläche 4 parallel verlaufenden Ebene angeordnet.

Durch die Zugvorrichtung 19 wird eine in Bildrichtung nach links gerichtete Kraft F auf die unteren Koppelmittel 26 aufgeprägt, die im Wesentlichen parallel zur Liegefläche 4 verläuft. Über die unteren Koppelmittel 26 wird die Kraft auf das erste Körperteil 27 des Patienten 28 übertragen. Gleichzeitig wirkt durch den Gegenhalter 20 eine identische Kraft F in entgegengesetzter Richtung. Diese Kraft F greift am oberen Koppelmittel 32 des Patienten 28 an, so dass der Patient 28 zwischen dem ersten Körperteil 27 und dem zweiten Körperteil 31 gestreckt wird.

Der Patient 28 verbleibt während des Streckvorgangs auf der Liegefläche 4 der Liegevorrichtung 2.

### Bezugszeichen:

1 - Extensionsvorrichtungsanordnung
2 - Liegevorrichtung
3 - Rahmen
4 - Liegefläche
5 - Fußteil
6 - Kopfteil
7 - unterer Längsbalken
8 - oberer Längsbalken
9 - erster Pfosten
10 - zweiter Pfosten
11 - Endbereich von 7, 8
12 - Winkelverbinder
13 - oberes Ende von 9, 10
14 - Balkenverbinder
15 - Hammerkopfschraube
16 - Stützpfosten
17 - Höhenabschnitt
18 - Halterung
19 - Zugvorrichtung
20 - Gegenhalter
21 - Endbereich von 20
22 - Öse von 20
23 - Verbindungsmittel
24 - Kombigerät aus Kraftmesser und Kraftbegrenzer
25 - Teilungselement
26 - unteres Koppelmittel
27 - erster Körperteil/Körperabschnitt
28 - Patient
29 - Ösen von 26
30 - Stabilisierungselement
31 - zweiter Körperteil/Körperabschnitt
32 - oberes Koppelmittel
33 - Ösen von 32
F Kraft

## Patentansprüche

1. Extensionsvorrichtungsanordnung (1) aufweisend eine Liegevorrichtung (2) mit einer Liegefläche (4) und einen die Liegevorrichtung (2) außen umgreifenden Rahmen (3), wobei der Rahmen (3) einen unteren Längsbalken (7) und einen lösbaren oberen Längsbalken (8) aufweist, die in ihren jeweiligen Endbereichen (11) über einen ersten Pfosten (9) und einen zweiten Pfosten (10) miteinander gekoppelt sind, wobei im Höhenabschnitt (17) der Liegefläche (4) an dem ersten Pfosten (9) eine Zugvorrichtung (19) und an dem zweiten Pfosten (10) ein Gegenhalter (20) angeordnet ist, so dass ein auf der Liegefläche (4) befindlicher Patient (28) jeweils an einem Körperteil (27, 31) oder Körperabschnitt (27, 31) über ein jeweiliges Koppelmittel (26, 32) mit der Zugvorrichtung (19) und dem Gegenhalter (20) verbunden ist, dergestalt, dass durch die Zugvorrichtung (19) zwischen den Körperteilen (27,31) oder Körperabschnitten (27, 31) eine im Wesentlichen parallel zur Liegefläche (4) wirkende Zugkraft (F) aufgebracht ist.

2. Anordnung nach Anspruch 1, wobei die Liegevorrichtung (2) als Bett, insbesondere als Krankenbett ausgebildet ist.

3. Anordnung nach einem der vorhergehenden Ansprüche, wobei der untere Längsbalken (7) und die Pfosten (9, 10) lösbar miteinander gekoppelt sind.

4. Anordnung nach einem der vorhergehenden Ansprüche, wobei der Rahmen (3) Standfüße (16) aufweist, die am unteren Längsbalken (7) angeordnet sind.

5. Anordnung nach einem der vorhergehenden Ansprüche, wobei das Koppelmittel (26, 32) zur Verbindung des Körperteils/Körperabschnitts (27, 31) mit der Zugvorrichtung (19) Manschetten aufweist, die über Verbindungsmittel (23), insbesondere Drahtseile, mit der Zugvorrichtung (19) gekoppelt sind.

6. Anordnung nach einem der vorhergehenden Ansprüche, wobei das Koppelmittel (26, 32) zur Verbindung des Körperteils/Körperabschnitts (27, 31) mit dem Gegenhalter (20) ein Geschirr aufweist, das über das Verbindungsmittel (23), insbesondere Drahtseile, mit dem Gegenhalter (20) gekoppelt ist.

7. Anordnung nach dem vorhergehenden Anspruch 6, wobei das Geschirr mindestens zwei Ösen (33) zur Kopplung mit dem Verbindungsmittel (23) aufweist.

8. Anordnung nach einem der vorhergehenden Ansprüche, wobei die Zugvorrichtung (19) aus einer Handwinde oder aus einer Elektroseilwinde gebildet ist.

9. Anordnung nach einem der vorhergehenden Ansprüche, wobei die Zugvorrichtung (19) höhenverstellbar am Pfosten (9) angeordnet ist und/oder die Zugvorrichtung (19) einen Kraftmesser (24) aufweist.

10. Anordnung nach einem der vorhergehenden Ansprüche, wobei die Zugvorrichtung (19) einen Kraftbegrenzer (24) aufweist.

11. Anordnung nach einem der vorhergehenden Ansprüche 5-10 wobei das Verbindungsmittel (23) der Zugvorrichtung (19) einen Längenindikator aufweist.

12. Anordnung nach einem der vorhergehenden Ansprüche, wobei der Gegenhalter (20) eine Traverse aufweist.

13. Anordnung nach einem der vorhergehenden Ansprüche, wobei der Gegenhalter (20) an seinen jeweiligen Endbereichen (21) über die Liegevorrichtung (2) hinausragt.

14. Anordnung nach einem der vorhergehenden Ansprüche, wobei der Gegenhalter (20) zwischen 0,8 und 1,3 m breit ist.

15. Anordnung nach einem der vorhergehenden Ansprüche, wobei der Rahmen (3) aus Holz, Metall, Leichtmetall oder Kunststoff ausgebildet ist.

## Claims

1. Extension device arrangement (1), having a lying device (2) with a lying surface (4) and a frame (3) surrounding the lying device (2) on the outside, wherein the frame (3) has a lower longitudinal beam (7) and a detachable upper longitudinal beam (8), which are coupled to one another in their respective end regions (11) via a first post (9) and a second post (10), wherein, in the vertical section (17) of the lying surface (4), a pulling device (19) is arranged on the first post (9) and a counter-holder (20) is disposed on the second post (10), such that a patient (28), located on the lying surface (4), is connected to the pulling device (19) and the counter-holder (20) at a body part (27, 31) or body section (27, 31) via a respective coupling means (26, 32), in such a way that a pulling force (F), acting essentially parallel to the lying surface (4), is applied by the pulling device (19) between the body parts (27, 31) or body sections (27, 31).

2. The arrangement according to claim 1, wherein the lying device (2) is configured as a bed, in particular as a hospital bed.

3. The arrangement according to any one of the preceding claims, wherein the lower longitudinal beam (7) and the posts (9, 10) are detachably coupled together.

4. The arrangement according to any one of the preceding claims, wherein the frame (3) has feet (16), arranged on the lower longitudinal beam (7).

5. The arrangement according to any one of the preceding claims, wherein the coupling means (26, 32) for connecting the body part/body section (27, 31) to the pulling device (19) has cuffs which are coupled to the pulling device (19) via connecting means (23), in particular wire ropes.

6. The arrangement according to any one of the preceding claims, wherein the coupling means (26, 32) for connecting the body part/body section (27, 31) to the counter-holder (20) has a harness which is coupled to the counter-holder (20) via the connecting means (23), in particular wire ropes.

7. The arrangement according to the preceding claim 6, wherein the harness has at least two eyelets (33) for coupling to the connecting means (23).

8. The arrangement according to any one of the preceding claims, wherein the pulling device (19) is formed from a hand winch or an electric cable winch.

9. The arrangement according to any one of the preceding claims, wherein the pulling device (19) is arranged on the post (9) in a height-adjustable manner and/or the pulling device (19) has a force gauge (24).

10. The arrangement according to any one of the preceding claims, wherein the pulling device (19) has a force delimiter (24).

11. The arrangement according to any one of the preceding claims 5-10, wherein the connecting means (23) of the pulling device (19) has a length indicator.

12. The arrangement according to any one of the preceding claims, wherein the counter-holder (20) has a cross member.

13. The arrangement according to any one of the preceding claims, wherein the counter-holder (20), at its respective end regions (21), protrudes beyond the lying device (2).

14. The arrangement according to any one of the preceding claims, wherein the counter-holder (20) is between 0.8 and 1.3 m wide.

15. The arrangement according to any one of the preceding claims, wherein the frame (3) is made of wood, metal, light metal or plastic.

## Revendications

1. Agencement du dispositif d'extension (1) présentant un dispositif de couchage (2) avec une surface de couchage (4) et un cadre (3) entourant extérieurement le dispositif de couchage (2), dans lequel le cadre (3) présente une poutre longitudinale inférieure (7) et une poutre longitudinale supérieure (8) amovible, qui sont couplées entre elles dans leurs zones d'extrémité (11) respectives par un premier montant (9) et un deuxième montant (10), dans lequel dans la section de hauteur (17) de la surface de couchage (4), un dispositif de traction (19) est disposé sur le premier montant (9) et un contre-appui (20) sur le deuxième montant (10), de sorte qu'un patient (28) se trouvant sur la surface de couchage (4) soit respectivement relié au dispositif de traction (19) et au contre-appui (20) au niveau d'une partie du corps (27, 31) ou d'une portion du corps (27, 31) par un moyen de couplage (26, 32) correspondant, de sorte qu'une force de traction (F) agissant sensiblement parallèlement à la surface de couchage (4) est appliquée par le dispositif de traction (19) entre les parties du corps (27, 31) ou les portions du corps (27, 31).

2. Agencement selon la revendication 1, dans lequel le dispositif de couchage (2) est conçu comme un lit, en particulier un lit médical.

3. Agencement selon l'une quelconque des revendications précédentes, dans lequel la poutre longitudinale inférieure (7) et les montants (9, 10) sont couplés entre eux de manière démontable.

4. Agencement selon l'une quelconque des revendications précédentes, dans lequel le cadre (3) présente des pieds d'appui (16) disposés sur la poutre longitudinale inférieure (7).

5. Agencement selon l'une quelconque des revendications précédentes, dans lequel le moyen de couplage (26, 32) pour relier la partie/portion du corps (27, 31) au dispositif de traction (19) présente des manchons qui sont couplés au dispositif de traction (19) par des moyens de liaison (23), notamment des câbles métalliques.

6. Agencement selon l'une quelconque des revendications précédentes, dans lequel le moyen de couplage (26, 32) pour la liaison de la partie/portion du corps (27, 31) avec le contre-appui (20) présente un harnais qui est couplé au contre-appui (20) via le moyen de liaison (23), en particulier des câbles métalliques.

7. Agencement selon la revendication 6 précédente, dans lequel le harnais présente au moins deux œillets (33) de couplage avec le moyen de liaison (23).

8. Agencement selon l'une quelconque des revendications précédentes, dans lequel le dispositif de traction (19) est constitué d'un treuil manuel ou d'un treuil électrique.

9. Agencement selon l'une quelconque des revendications précédentes, dans lequel le dispositif de traction (19) est disposé de manière réglable en hauteur sur le montant (9) et/ou le dispositif de traction (19) présente un dynamomètre (24).

10. Agencement selon l'une quelconque des revendications précédentes, dans lequel le dispositif de traction (19) présente un limiteur de force (24).

11. Agencement selon l'une des revendications 5 à 10 précédentes, dans lequel le moyen de liaison (23) du dispositif de traction (19) présente un indicateur de longueur.

12. Agencement selon l'une quelconque des revendications précédentes, dans lequel le contre-appui (20) présente une traverse.

13. Agencement selon l'une quelconque des revendications précédentes, dans lequel le contre-appui (20) dépasse du dispositif de couchage (2) à ses zones d'extrémité (21) respectives.

14. Agencement selon l'une quelconque des revendications précédentes, dans lequel le contre-appui (20) a une largeur comprise entre 0,8 et 1,3 m.

15. Agencement selon l'une quelconque des revendications précédentes, dans lequel le cadre (3) est réalisé en bois, en métal, en métal léger ou en plastique.
